# EUROPÄISCHE PATENTSCHRIFT

(11) **EP 1 056 399 B1**
(45) Veröffentlichungstag und Bekanntmachung des Hinweises auf die Patenterteilung: **13.12.2006**
(21) Anmeldenummer: 99906048.6
(22) Anmeldetag: 14.01.1999
(51) Int. Cl.: A61B 17/28

(54) **INSTRUMENT ZUM EINSATZ BEI ENDOSKOPISCHEN EINGRIFFEN**
INSTRUMENT USED FOR ENDOSCOPIC INTERVENTIONS
INSTRUMENT A UTILISER LORS D'OPERATIONS ENDOSCOPIQUES

(30) Priorität: 14.01.1998 DE 19800917
(43) Veröffentlichungstag der Anmeldung: 06.12.2000
(73) Patentinhaber: Karl Storz GmbH & Co. KG, 78532 Tuttlingen (DE)
(72) Erfinder: MANHES, Hubert, F-003200 Vichy (FR)
(74) Vertreter: Popp, Eugen
(86) Internationale Anmeldenummer: PCT/DE1999/000057
(87) Internationale Veröffentlichungsnummer: WO 1999/035981

(56) Entgegenhaltungen:
- DE-A- 19 627 992
- GB-A- 1 033 708
- US-A- 5 234 443
- US-A- 5 474 057
- US-A- 5 511 564

## Beschreibung

Die Erfindung bezieht sich auf ein Instrument zum Einsatz bei endoskopischen Eingriffen.

### Stand der Technik

Aus der US-PS 5,474,057 ist ein Instrument bekannt, von dem bei der Formulierung des Oberbegriffs der Patansprüche 1 und 2 ausgegangen wurde. Zur Realisierung der mit der vorliegenden Erfindung durchführbaren Eingriffe ist das Instrument aus der US-PS 5,474,057 jedoch aus folgenden Gründen nicht oder nur bedingt geeignet. In diesem Instrument sind die Achsen der Maulteile der Zangenelemente parallel zu den Schwenkachsen der Zangenelemente ausgeführt. Beide Zangenmäuler können nur als ganzes geschwenkt werden.

Aus der Druckschrift DE 43 24 254 C1 ist ein chirurgisches Instrument für endoskopische Operationen bekannt, das einen Schaft aufweist. In dem Schaft sind drei Kanäle vorgesehen, die an den Spitzen eines gleichschenkligen Dreiecks angeordnet sind. In zwei der Kanäle können Zangen oder dergleichen eingesetzt werden, deren Zangenmaul geschwenkt werden kann. In den dritten Kanal soll eine Optik eingesetzt werden.
Die Orientierung der Zangen zueinander kann dabei beliebig sein, da die Zangen unter einem beliebigen Drehwinkel in den jeweiligen Kanal eingesetzt werden können.

Damit läßt sich das bekannte chirurgische Instrument zwar an nahezu beliebige Operationssituationen anpassen, für bestimmte Aufgaben, bei denen genau festgelegte Arbeitsabläufe erforderlich sind, ist jedoch der Justieraufwand sehr hoch;

So ist es bei einer Reihe von endoskopischen Eingriffen erforderlich, zwei Gewebeteile, die voneinander getrennt sind, "zusammenzuziehen", in diesem Zustand festzuhalten und anschließend beispielsweise durch einen Näh- oder Klebevorgang miteinander zu verbinden.

Beispiele für derartige Eingriffe sind die Operation von gerissenen Bändern in einem Gelenk, wie beispielsweise dem Kniegelenk, oder die invitro-Fertilisation der Eileiter. Ein weiteres Beispiel ist in dem Artikel von D.J. TIBBS et al. "Arterial Replacement with Minimal Interruption of Blood-Flow, erschienen in "The Lancet, 1958, pp. 292 bis 294" beschrieben.

Die Durchführung dieser Eingriffe ist mit dem aus der DE 43 24 254 C1 bekannten Instrument nicht möglich, da die Schwenkachsen der Zangenmaul-Teile und des Zangenmauls parallel zueinander angeordnet sind. Damit gestatten die beiden unabhängig voneinander in einen Schaft mit mehreren Kanälen eingeführten abwinkelbaren Zangen keine koordinierte Bewegung im Sinne einer gezielten Annäherung von Teilen, die von den beiden Zangenmäuler gehalten werden. Zudem sind die einzelnen Kanäle an den Spitzen eines gleichseitigen Dreiecks angeordnet, so daß die Manipulation des zusammengezogenen Gewebeteils durch ein in den dritten Kanal eingeführtes Instrument erschwert wird.

Mit herkömmlichen - getrennten - Instrumenten erfordern derartige Eingriffe nicht nur viel Zeit, sondern auch ein großes manuelles Geschick des den Eingriff ausführenden Arztes, da dieser mehrere getrennt in den menschlichen Körper eingeführte Instrumente handhaben und ihre Bewegung koordinieren muß.

### Darstellung der Erfindung

Der Erfindung liegt die Aufgabe zugrunde, ein Instrument zum Einsatz bei endoskopischen Eingriffen anzugeben, durch das beispielsweise das "Zusammenziehen" zweier Gewebeteile, die voneinander getrennt sind, das Festhalten der Gewebeteile im zusammengezogenen Zustand und das anschließende Manipulieren, also beispielsweise das Verbinden durch einen Näh- oder Klebevorgang vereinfacht wird. Das erfindungsgemäße Instrument soll darüber hinaus einen einfachen Aufbau haben und nur einen geringen Vorbereitungs- bzw. Justieraufwand erfordern.

Die erfindungsgemäße Lösung dieser Aufgabe ist in Patentanspruch 1 angegeben. Weiterbildungen der Erfindung sind Gegenstand der Ansprüche 2 folgende.

Erfindungsgemäß wird von einem Instrument zum Einsatz bei endoskopischen Eingriffen ausgegangen, das einen Schaft aufweist, der an seinem distalen Ende zwei unabhängig voneinander durch Betätigungselemente betätigbare Zangenelemente aufweist, von denen eines um eine zur Längsachse des Instruments wenigstens annähernd senkrechte Achse schwenkbar ist, und der wenigstens einen weiteren Kanal aufweist.

Zur Vermeidung von Fehlbedienungen sind bei dem erfindungsgemäßen Instrument die Manipulationsmöglichkeiten für die Bewegung der Zangenmäuler als Einheit eingeschränkt:

Bei der in Anspruch 1 angegebenen Ausführungsform ist lediglich ein Zangenelement schwenkbar, während das andere Zangenelement nicht schwenkbar ist.

Damit eignet sich das erfindungsgemäße Instrument insbesondere für die Durchführung spezieller Aufgaben, wie beispielsweise das Zusammenziehen von Gewebeteilen.

Hierzu ist das Instrument derart ausgeführt, dass das oder die schwenkbaren Maulteile jedes Zangenelements um Achsen schwenkbar sind, von denen jede senkrecht zur Achse steht, um die das eine Zangenelement verschwenkbar ist. Diese Orientierung, die der aus der DE 43 24 254 C1 bekannten Orientierung zwischen Zangenmaul und Schwenkachse entgegengesetzt ist, ermöglicht das Zusammenziehen auch empfindlicher Gewebeteile, da sich die Maulteile beim Greifen in etwa senkrecht zu der Richtung bewegen, in der die Gewebeteile durch Schwenken des schwenkbaren Zangenelements bewegt werden.

Wenn die distale Austrittsöffnung eines durchgehenden Kanals zwischen den Mäulern der beiden Zangenelemente vorgesehen ist, und der durchgehende Kanal so ausgebildet ist, dass in ihn (u. a.) ein Operationsinstrument eingesetzt werden kann, das mit den Mäulern der beiden Zangenelemente zusammenwirkt, ist beispielsweise folgende Vorgehensweise zur Verbindung getrennter Gewebeteile möglich:

Das erfindungsgemäße Instrument wird in den Hohlraum eingeführt, in dem sich die zu verbindenden Gewebeteile befinden. Die freien Enden der Gewebeteile, also beispielsweise der Eileitertuben werden mit dem

Maul jeweils eines Zangenelements des Instruments ergriffen. Anschließend wird das Zangenmaul einer Zange in einer Richtung senkrecht zur Längsachse des Instruments auf die andere Zange zu bewegt. Da die beiden Zangenelemente des Instruments die Gewebeteile festhalten, wird das eine freie Ende eines der beiden zu verbindenden Gewebeteile auf das Ende des anderen Gewebeteils zu bewegt. Sobald sich die freien Enden in einer Position befinden, in der die Ausführung des Verbindungsvorgangs möglich ist, führt der behandelnde Arzt den Verbindungsvorgang aus. Während des Verbindungsvorgangs und gegebenenfalls auch noch danach werden die zu verbindenden Teile mit dem erfindungsgemäßen Instrument festgehalten.

Selbstverständlich kann das erfindungsgemäße Instrument auch für andere Behandlungs- bzw. Bearbeitungsvorgänge im menschlichen oder tierischen Körper oder bei technischen Einsätzen verwendet werden.

Weiterhin ist es von Vorteil, wenn am proximalen Ende des Instruments für das schwenkbare Zangenelement ein Schwenk-Betätigungselement vorgesehen ist, durch dessen Betätigung der Schwenkwinkel und damit der Abstand zwischen den Zangenelementen in Richtung der Querachse geändert wird. Die Änderung des Schwenkwinkels hat dabei keinen Einfluss auf die Relativ-Stellung der Maulteile dieses Zangenelements zueinander. Damit ist es möglich, die zu verbindenden freien Gewebeteile präzise und ohne Schädigung des Gewebes zusammenzuführen.

Es ist auch eine Weiterbildung möglich, bei der die Zangenelemente in Art bekannter Zangen aufgebaut sind bzw. bekannte Zangen sind, die als Einheit in einen Schaft eingesetzt sind. Diese Ausbildung vereinfacht sowohl die Herstellung, die Lagerhaltung beim Hersteller als auch die Reinigung des erfindungsgemäßen Instruments.

Dabei ist es ferner von Vorteil, wenn der Schaft in Art eines bekannten Trokars oder eines Laparoskops ausgebildet ist. Dieser Trokarschaft kann beispielsweise dann, wenn Eileiter-Tuben vernäht werden sollen, einen Außendurchmesser von 10 mm bis 13 mm haben.

Weiterhin ist es bevorzugt, wenn die Betätigungselemente für die Maulteile der Zangen Griffstücke - wie Scherengriffe, Zangengriffe oder dgl. - sind, die in die Stellung vorgespannt sind, in der das jeweilige Zangenmaul geschlossen ist. Diese Ausbildung hat den Vorteil, dass der Arzt beim Zusammenziehen der Gewebeteile die Maulteile der beiden Zangen nicht durch Festhalten der Griffstücke im geschlossenen Zustand halten muss.

Die Variation des Abstandes zwischen den beiden Zangenelementen kann prinzipiell auf die verschiedensten Arten erfolgen. Beispielsweise ist es möglich, das schwenkbare Zangenelement an einer elastischen und nach außen gebogenen Halterung zu halten. Mittels einer Linearführung oder einer Hülse wird dann das Zangenelement "nach innen gedrückt" bzw. der Anlenkpunkt verschoben.

Einen besonders großen Verstellbereich erhält man - gerade im Hinblick auf den begrenzten Durchmesser von Endoskopen - durch die in den Ansprüchen folgende angegebenen Merkmale, die verschiedene Möglichkeiten für die Verschwenkung des distalen Endes der Zangenelemente bzw. Zangen als Einheit angeben:

Gemäß Anspruch 7 sind die Zangenmäuler Bestandteil an sich bekannter flexibler Zangen, deren distales Ende in ebenfalls an sich bekannter Weise abwinkelbar ist.

Die Schwenk- bzw. Abwinkelbewegung der Zangenmäuler kann auf die Verschiedensten Arten hervorgerufen werden:

So können gemäß Anspruch 8 Übertragungselemente, wie beispielsweise Seilzüge, Schub- bzw. Zugstangen vorgesehen sein, die die Bewegung der Betätigungselemente auf die Zangenmäuler übertragen. Weiterhin ist es möglich, dass distal angeordnete Actuatoren vorgesehen sind, die die Schwenk- bzw. Abwinkelbewegung erzeugen (Anspruch 9). Die Actuatoren können gemäß Anspruch 10 insbesondere elektrisch betätigte Mikrostellelemente, wie Mikromotoren sein.

Weiterhin ist es möglich, als Zange wenigstens eine starre Zange zu verwenden, deren distales Ende als Einheit abgewinkelt werden kann, und deren Maulteile über eine Stange oder dgl. mit einem proximal angeordneten Betätigungselement verbunden sind (Anspruch 11). Eine derartige Zange ist beispielsweise in der WO 97/49342 beschrieben.

Dabei ist es gemäß Anspruch 12 bevorzugt, wenn ein Ausgleichsmechanismus vorgesehen ist, der eine Änderung der relativen Winkelstellung der beiden Maulteile des Mauls beim Abwinkeln bzw. Schwenken des distalen Endes als Einheit verhindert, da dann beim Ändern des Abstandes der beiden Zangen das Gewebe weder geschädigt werden kann noch aus dem jeweiligen Maulteil herausgleiten kann.

Bei Verwendung einer starren Zange ist es weiterhin bevorzugt, wenn die Stange in an sich bekannter Weise im Bereich der Abwinkelung flexibel ausgebildet ist und auf Zug oder Druck das Maul schließt.

Beim Einsatz einer "starren Zange" ist es weiterhin bevorzugt, wenn ein Stellelement und insbesondere eine Stellschraube vorgesehen ist, durch dessen bzw. deren Betätigung das distale Ende abwinkelbar bzw. schwenkbar ist.

Die Stellschraube kann unter einem Winkel von 90° zur Längsachse des Instruments oder konzentrisch zur Längsachse des Instruments angeordnet sein.

Die Handhabung des erfindungsgemäßen Instruments wird weiter vereinfacht, wenn bestimmte Winkelstellungen der beiden Zangen indexiert sind, oder wenn die Stellschraube eine Rastung bei bestimmten Winkelstellungen aufweist.

In den zusätzlichen Kanal können die verschiedensten Instrumente eingesetzt werden:

Beispielsweise ist es möglich, eine Nähvorrichtung oder eine Klebevorrichtung einzusetzen.

Insbesondere dann, wenn distale und proximale Eileiter stümpfe nach Einführung eines Katheders miteinander verbunden werden sollen, ist es von Vorteil, wenn die Klebevorrichtung ein Katheder ist, der das Aufbringen eines Fibrinklebers gestattet.

Die Zangenmäuler des erfindungsgemäßen Instruments können prinzipiell in jeder bekannten Ausführungsform ausgebildet sein:

Besonders vorteilhaft ist es jedoch, wenn die Zangenmäuler durch jeweils zwei Greifbacken gebildet werden. Diese Greifbacken können mit Zähnen versehen sein, so daß die jeweiligen Gewebeteile sicher gegriffen werden können.

Dabei kann nach die Form der Greifbacken den zusammenzufügenden Gewebeteilen angepaßt sein, so daß ein großflächiger Formschluß erreicht wird.

Die Anpassung an unterschiedliche Operationsbedingungen wird dadurch erleichtert, daß die Zangenmäuler zusätzlich in Richtung der Instrumenten-Längsachse gegenüber dem Instrumentenkörper verschiebbar sind. Die Verschiebung jedes Zangenmauls kann dabei unabhängig von dem anderen Zangenmaul erfolgen.

Durch die Verschiebbarkeit in Richtung der Längsachse kann u.a. der Winkelversatz ausgeglichen werden, der sich durch die Verschwenkung ergibt. Gegebenenfalls ist auch eine Zwangsführung zum Ausgleich des Winkelversatzes möglich.

### Kurze Beschreibung der Zeichnung

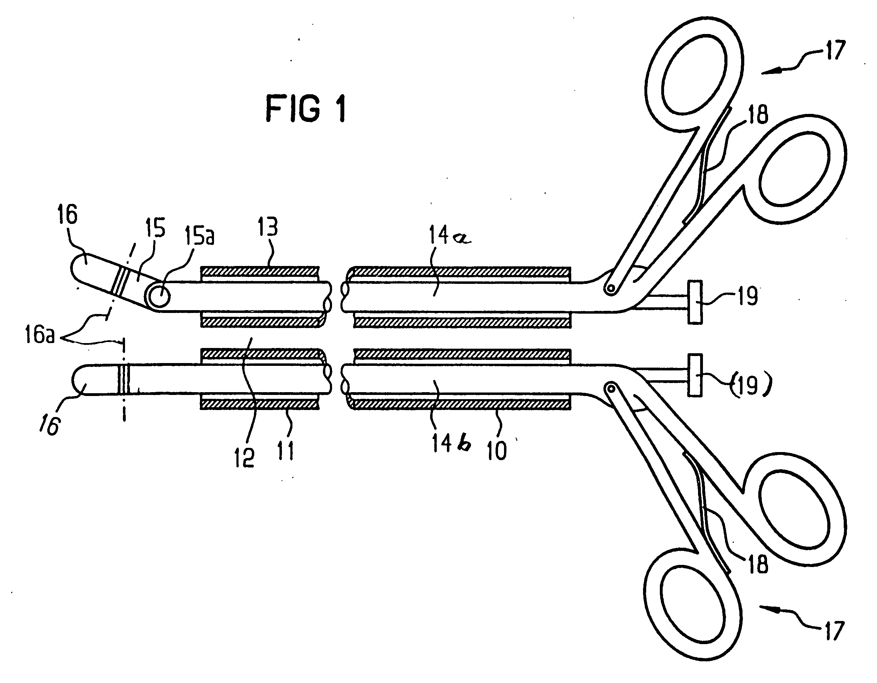

- Fig. 1: eine teilweise geschnittene Seitenansicht eines Ausführungsbeispiels eines erfindungsgemäßen Instruments,

### Darstellung des Ausführungsbeispiels

Fig. 1 zeigt ein Ausführungsbeispiel der Erfindung. Dieses Instrument weist einen Schaft 10 auf, in dem drei nebeneinanderliegende Kanäle 11 bis 13 vorgesehen sind. In die beiden äußeren Kanäle ist jeweils eine Zange 14a bzw. 14b eingesetzt. Das distale Ende 15 der Zange 14a ist als Einheit um eine Schwenkachse 15a schwenkbar. Das distale Ende 15 der Zange 14b ist starr.

Das distale Ende 15 beider Zangen umfaßt ferner Maulteile 16 der beiden Zangen; die Maulteile 16 sind um eine Achse 16a schwenkbar, die senkrecht auf der Achse 15a steht (in der Draufsicht in Figur 1 ist lediglich eines der beiden Maulteile zu sehen.

Zur Betätigung der Maulteile sind in an sich bekannter Weise Scherengriffe 17 vorgesehen, die durch eine Feder 18 in die Stellung vorgespannt sind, in der die Maulteile 16 geschlossen sind. Zur Abwinkelung des distalen Endes 15 der Zange 14a als Einheit ist ein Betätigungselement 19 vorgesehen, das koaxial zur Zangen-Längsachse angeordnet ist.

Die Betätigungselemente 17 und 19 sind über Stangen oder Seilzüge mit den jeweiligen distalen Elementen verbunden.

Bevorzugt weist das Betätigungselement 19 eine Indexierung oder Rastung auf, so daß bestimmte Abwinkelungen der Elemente 15 leicht eingestellt werden können.

Zusätzlich sind die Zangen 14 in ihren jeweiligen Kanälen in Richtung ihrer Längsachse verschiebbar, so daß umfangreiche Manipulationen möglich sind.

Die Form der insbesondere mit Zähnen versehenen Greifbacken ist dem jeweiligen Anwendungsfall angepaßt.

In den mittleren Kanal 12 kann ein Instrument, wie beispielsweise ein Katheder zum Aufbringen eines Klebers, eine Nähvorrichtung oder auch eine weitere Zange eingesetzt werden.

Durch die erfindungsgemäße Ausbildung ist die Durchführung der verschiedensten Vorgänge möglich, ohne daß die Gefahr besteht, daß die Bedienungsperson durch zu umfangreiche Verstellmöglichkeiten überfordert wäre.

## Patentansprüche

1. Instrument zum Einsatz bei endoskopischen Eingriffen, mit einem Schaft (10), der
- an seinem distalen Ende zwei unabhängig voneinander durch Betätigungselemente (17) betätigbare Zangenelemente aufweist, die verschwenkbare Maulteile (16) aufweisen,
- von denen eines um eine zur Längsachse des Instruments wenigstens annähernd senkrechte Achse (15a) schwenkbar ist, und
- der wenigstens einen durchgehenden Kanal (12) zwischen den Mäulern der beiden Zangenelemente aufweist,
**dadurch gekennzeichnet, dass**
das andere Zangenelement nicht schwenkbar ist, und dass das oder die schwenkbaren Maulteile (16) jedes Zangenelements um Achsen (16a) schwenkbar sind, von denen jede senkrecht zur Achse (15a) steht, um die das eine Zangenelement verschwenkbar ist.

2. Instrument nach Anspruch 1,
**dadurch gekennzeichnet, dass**
am proximalen Ende des Instruments für das schwenkbare Zangenelement ein Schwenk-Betätigungselement (19) vorgesehen ist, dessen Betätigung die Schwenkstellung des Zangenelements ändert, ohne dass die Relativ-Stellung der beiden Maulteile (16) dieser Zange zueinander geändert wird.

3. Instrument nach einem der Ansprüche 1 bis 2,
**dadurch gekennzeichnet, dass**
die Zangenelemente Bestandteile jeweils einer Zange sind, die als Einheit in einen Kanal (11, 13) des Schafts (10) eingesetzt ist.

4. Instrument nach Anspruch 3,
**dadurch gekennzeichnet, dass**
die Zangen (14a, 14b) in dem jeweiligen Kanal (11, 13) in Richtung der Längsachse des Schafts (10) verschiebbar sind.

5. Instrument nach einem der Ansprüche 1 bis 4,
**dadurch gekennzeichnet, dass**
die Betätigungselemente (17) für die Maulteile (16) der Zangen Griffstücke sind, die in die Stellung vorgespannt sind, in der das Zangenmaul geschlossen ist.

6. Instrument nach einem der Ansprüche 1 bis 5,
**dadurch gekennzeichnet, dass** die schwenkbare
Zange in Art einer flexiblen Zange aufgebaut ist, deren distales Ende (15) abwinkelbar ist.

7. Instrument nach einem der Ansprüche 1 bis 6,
**dadurch gekennzeichnet, dass**
Übertragungselemente vorgesehen sind, die die Schwenk- bzw. Abwinkelbewegung des bzw. der Zangenmäuler (16) erzeugen.

8. Instrument nach einem der Ansprüche 1 bis 7,
**dadurch gekennzeichnet, dass**
distal angeordnete Actuatoren vorgesehen sind, die die Schwenk- bzw. Abwinkelbewegung des bzw. der Zangenmäuler (16) erzeugen.

9. Instrument nach Anspruch 8,
**dadurch gekennzeichnet, dass**
die Actuatoren elektrisch betätigte Mikrostellelemente sind.

10. Instrument nach einem der Ansprüche 1 bis 9,
**dadurch gekennzeichnet, dass** die schwenkbare
Zange eine starre Zange ist, deren distales Ende (15) um eine Achse (15a) abwinkelbar oder durch Verschieben einer Hülse oder eines vergleichbaren Bauelementes gegen eine elastische Vorspannung verschiebbar ist, und deren Maulteile (16) über eine Stange oder ein damit vergleichbares Funktionselement mit einem proximal angeordneten Betätigungselement (19) verbunden sind.

11. Instrument nach einem der Ansprüche 1 bis 10,
**dadurch gekennzeichnet, dass**
ein Ausgleichsmechanismus vorgesehen ist, der eine Änderung der relativen Winkelstellung der beiden Maulteile (16) des Mauls beim Abwinkeln bzw. Schwenken des distalen Endes (15) als Einheit verhindert.

12. Instrument nach Anspruch 10 oder 11,
**dadurch gekennzeichnet, dass**
die Stange im Bereich der Abwinklung flexibel ausgebildet ist.

13. Instrument nach einem der Ansprüche 10 bis 12,
**dadurch gekennzeichnet, dass**
die Stange auf Zug oder Druck das Maul schließt.

14. Instrument nach einem der Ansprüche 10 bis 12,
**dadurch gekennzeichnet, dass**
ein Stellelement (19) vorgesehen ist, durch dessen Betätigung das distale Ende (15) abwinkelbar bzw. schwenkbar ist.

15. Instrument nach Anspruch 14,
**dadurch gekennzeichnet, dass**
das Stellelement (19) eine Stellschraube ist.

16. Instrument nach Anspruch 15,
**dadurch gekennzeichnet, dass**
die Stellschraube unter einem Winkel von 90° zur Längsachse des Instruments angeordnet ist.

17. Instrument nach Anspruch 15,
**dadurch gekennzeichnet, dass**
die Stellschraube konzentrisch zur Längsachse des Instruments angeordnet ist.

18. Instrument nach einem der Ansprüche 1 bis 17,
**dadurch gekennzeichnet, dass**
bestimmte Winkelstellungen der beiden Zangen (14a, 14b) indexiert sind, oder
dass die Stellschraube eine Rastung bei bestimmten Winkelstellungen aufweist.

19. Instrument nach einem der Ansprüche 1 bis 18,
**dadurch gekennzeichnet, dass**
das Instrument in einen Trokarschaft einsetzbar ist.

20. Instrument nach Anspruch 19,
**dadurch gekennzeichnet, dass**
der Trokarschaft einen Außendurchmesser von 10 bis 13 mm hat.

21. Instrument nach einem der Ansprüche 1 bis 20,
**dadurch gekennzeichnet, dass**
in den durchgehenden Kanal (12) eine Nähvorrichtung oder eine Klebevorrichtung einsetzbar ist.

22. Instrument nach Anspruch 21,
**dadurch gekennzeichnet, dass**
die Klebevorrichtung ein Katheder ist, der das Aufbringen eines Fibrinklebers gestattet.

23. Vorrichtung nach einem der Ansprüche 1 bis 22,
**dadurch gekennzeichnet, dass**
die Zangenmäuler (16) durch jeweils zwei Greif- oder Scherenbacken gebildet werden.

24. Vorrichtung nach Anspruch 23,
**dadurch gekennzeichnet, dass**
die Greifbacken mit Zähnen versehen sind.

25. Vorrichtung nach Anspruch 23 oder 24,
**dadurch gekennzeichnet, dass**
die Form der Greifbacken den zusammenzufügenden Gewebeteilen angepasst ist.

26. Vorrichtung nach einem der Ansprüche 1 bis 25,
**dadurch gekennzeichnet, dass**
die Zangenmäuler (16) zusätzlich in Richtung der Instrumenten-Längsachse verschiebbar sind.

27. Vorrichtung nach Anspruch 26,
**dadurch gekennzeichnet, dass**
jedes Zangenmaul (16) unabhängig von dem anderen Zangenmaul (16) verschiebbar ist.

## Claims

1. An instrument for employment in endoscopic operations, with a shaft (10) which
- comprises at its distal end two forceps elements which may be operated independent from one another by actuation elements (17), which comprise pivotable mouth parts (16),
- one of which is pivotable about an axis (15a) which is at least approximately perpendicular to the longitudinal axis of the instrument, and
- which comprises at least one continuous channel (12) between the mouths of the two forceps elements,
**characterised in that**
the other forceps element is not pivotable and the pivotable mouth part(s) (16) of each forceps element is (are) pivotable about axes (16a) each of which being perpendicular to the axis (15a) about which the one forceps element is pivotable.

2. The instrument according to Claim 1,
**characterised in that**
a the proximal end of the instrument, a pivoting actuation element (19) is provided for the pivotable forceps element, whose actuation changes the pivot position of the forceps element without the relative position of the two mouth parts (16) of said forceps being changed.

3. The instrument according to one of Claims 1 to 2,
**characterised in that**
the forceps elements are parts of one forceps each which is inserted as a unit into a channel (11, 13) of the shaft (10).

4. The instrument according to Claim 3,
**characterised in that**
the forceps (14a, 14b) are movable within the respective channel (11, 13) in the direction of the longitudinal axis of the shaft (10).

5. The instrument according to one of Claims 1 to 4,
**characterised in that**
the actuation elements (17) for the mouth parts (16) of the forceps are handle parts which are biased to the position in which the forceps mouth is closed.

6. The instrument according to one of Claims 1 to 5,
**characterised in that**
the pivotable forceps are constructed like flexible forceps whose distal end (15) may be bent.

7. The instrument according to one of Claims 1 to 6,
**characterised in that**
transmission elements are provided which generate the pivoting or bending movement, respectively, of the forceps mouth or mouths (16), respectively.

8. The instrument according to one of Claims 1 to 7,
**characterised in that**
distally arranged actuators are provided which generate the pivoting or bending movement, respectively, of the forceps mouth or mouths (16), respectively.

9. The instrument according to Claim 8,
**characterised in that**
the actuators are electrically operated micro-actuators.

10. The instrument according to one of Claims 1 to 9,
**characterised in that**
the pivotable forceps are rigid forceps whose distal end (15) may be bent about an axis (15a) or moved by adjusting a sleeve or a similar components against an elastic bias, and whose mouth parts (16) are connected via a rod or a similar functional element with a proximally arranged actuation element (19).

11. The instrument according to one of Claims 1 to 10,
**characterised in that**
a compensation mechanism is provided which prevents a change of the relative angular position of the two mouth parts (16) of the mouth upon bending or pivoting, respectively, of the distal end (15) as a unit.

12. The instrument according to Claim 10 or 11,
**characterised in that**
the rod is designed flexible in the area of the bend.

13. The instrument according to one of Claims 10 to 12,
**characterised in that**
the bar closes the mouth upon tension or pressure.

14. The instrument according to one of Claims 10 to 12,
**characterised in that**
an actuator (19) is provided through the actuation of which the distal end (15) may be bent or pivoted, respectively.

15. The instrument according to Claim 14,
**characterised in that**
the actuator (19) is a set screw.

16. The instrument according to Claim 15,
**characterised in that**
the set screw is arranged at an angle of 90° to the longitudinal axis of the instrument.

17. The instrument according to Claim 15,
**characterised in that**
the set screw is arranged concentrically with the longitudinal axis of the instrument.

18. The instrument according to one of Claims 1 to 17,
**characterised in that**
certain angular positions of the two forceps (14a, 14b) are indexed, or that the set screw comprises a detent at certain angular positions.

19. The instrument according to one of Claims 1 to 18,
**characterised in that**
the instrument may be inserted into a trocar shaft.

20. The instrument according to Claim 19,
**characterised in that**
the trocar shaft has an outer diameter of 10 to 13 mm.

21. The instrument according to one of Claims 1 to 20,
**characterised in that**
a sewing device or an adhesive device may be inserted into the continuous channel (12).

22. The instrument according to Claim 21,
**characterised in that**
the adhesive device is a catheter which allows the application of a fibrin adhesive.

23. An apparatus according to one of Claims 1 to 22,
**characterised in that**
the forceps mouths (16) are formed by two gripping or scissors jaws each.

24. The apparatus according to Claim 23,
**characterised in that**
the gripping jaws are provided with teeth.

25. The apparatus according to Claim 23 or 24,
**characterised in that**
the shape of the gripping jaws is adapted to the tissue parts to be joined.

26. The according to one of Claims 1 to 25,
**characterised in that**
the forceps mouths (16) are additionally movable in the direction of the instrument's longitudinal axis.

27. The apparatus according to Claim 26,
**characterised in that**
each forceps mouth (16) is movable independent of the other forceps mouth (16).

## Revendications

1. Instrument à utiliser pour des interventions endoscopiques, comprenant une tige (10) :
-- qui comporte à son extrémité distale deux éléments de pince, susceptibles d'être actionnés indépendamment l'un de l'autre par des éléments d'actionnement (17), qui comprennent des pièces de mâchoire (16) pivotantes,
-- dont l'une est capable de pivoter autour d'un axe (15a) au moins approximativement perpendiculaire à l'axe longitudinal de l'instrument, et
-- qui présente au moins un canal (12) traversant entre les mâchoires des deux éléments de pince,
**caractérisé en ce que**
l'autre élément de pince n'est pas susceptible de pivoter, et **en ce que** la ou les pièce(s) de mâchoire pivotante(s) (16) de chaque élément de pince et/sont capable(s) de pivoter autour d'axes (16a) qui sont chacun perpendiculaire à l'axe (15a) autour duquel l'un des éléments de pince est capable de pivoter.

2. Instrument selon la revendication 1,
**caractérisé en ce que**, à l'extrémité proximale de l'instrument, il est prévu pour l'élément de pince capable de pivoter un élément d'actionnement de pivotement (19) dont l'actionnement modifie la position en pivotement de l'élément de pince sans modifier la position relative des deux pièces de mâchoire (16) de cette pince l'une par rapport à l'autre.

3. Instrument selon l'une des revendications 1 et 2,
**caractérisé en ce que** les éléments de pince sont des composants respectifs d'une pince qui est mise en place sous forme d'une unité dans un canal (11, 13) de la tige (10).

4. Instrument selon la revendication 3,
**caractérisé en ce que** les pinces (14a, 14b) sont déplaçables en translation dans le canal respectif (11, 13) en direction de l'axe longitudinal de la tige (10).

5. Instrument selon l'une des revendications 1 à 4,
**caractérisé en ce que** les éléments d'actionnement (17) pour les pièces de mâchoire (16) des pinces sont des éléments de préhension qui sont précontraints vers la position dans laquelle l'embouchure de pince est fermée.

6. Instrument selon l'une des revendications 1 à 5,
**caractérisé en ce que** la pince pivotante est réalisée à la manière d'une pince flexible, dont l'extrémité distale (15) peut être déviée sous un angle.

7. Instrument selon l'une des revendications 1 à 6,
**caractérisé en ce qu'**il est prévu des éléments de transmission qui engendrent le mouvement de pivotement ou le mouvement de mise en angle de la ou des mâchoires de pince (16).

8. Instrument selon l'une des revendications 1 à 7,
**caractérisé en ce qu'**il est prévu des actionneurs agencés en situation distale, qui engendrent le mouvement de pivotement ou le mouvement de mise en angle de la ou des mâchoires de pince (16).

9. Instrument selon la revendication 8,
**caractérisé en ce que** les actionneurs sont des éléments de micro-positionnement à actionnement électrique.

10. Instrument selon l'une des revendications 1 à 9,
**caractérisé en ce que** la pince pivotante est une pince rigide dont l'extrémité distale (15) peut être mise sous un angle autour d'un axe (15a) ou peut être déplacée en translation à l'encontre d'une précontrainte élastique par translation d'une douille ou d'un élément comparable, et dont les pièces de mâchoire (16) sont reliées à un élément d'actionnement (19) agencé en situation proximale, via une tringle ou un élément fonctionnel comparable à une tringle.

11. Instrument selon l'une des revendications 1 à 10,
**caractérisé en ce qu'**il est prévu un mécanisme de compensation qui empêche une modification de la position angulaire relative des deux pièces de mâchoire (16) de l'embouchure à titre d'unité lors de la mise en angle ou du pivotement de l'extrémité distale (15).

12. Instrument selon la revendication 10 ou 11,
**caractérisé en ce que** la tringle est réalisée flexible dans la zone de la mise en angle.

13. Instrument selon l'une des revendications 10 à 12,
**caractérisé en ce que** la tringle ferme l'embouchure par traction ou par poussée.

14. Instrument selon l'une des revendications 10 à 12,
**caractérisé en ce qu'**il est prévu un élément de positionnement (19), par actionnement duquel l'extrémité distale (15) peut être mise en angle ou pivotée.

15. Instrument selon la revendication 14,
**caractérisé en ce que** l'élément de positionnement (19) est une vis de positionnement.

16. Instrument selon la revendication 15,
**caractérisé en ce que** la vis de positionnement et agencée sous un angle de 90° par rapport à l'axe longitudinal de l'instrument.

17. Instrument selon la revendication 15,
**caractérisé en ce que** la vis de positionnement est agencée concentrique à l'axe longitudinal de l'instrument.

18. Instrument selon l'une des revendications 1 à 17,
**caractérisé en ce que** certaines positions angulaires des deux pinces (14a, 14b) sont indexées, ou **en ce que** la vis de positionnement présente un cran à certaines positions angulaires.

19. Instrument selon l'une des revendications 1 à 18,
**caractérisé en ce que** l'instrument peut être utilisé dans une tige de trocart.

20. Instrument selon la revendication 19,
**caractérisé en ce que** la tige de trocart a un diamètre extérieur de 10 à 13 mm.

21. Instrument selon l'une des revendications 1 à 20,
**caractérisé en ce qu'**un dispositif de suture ou un dispositif de collage est susceptible d'être mis en place dans le canal traversant (12).

22. Instrument selon la revendication 21,
**caractérisé en ce que** le dispositif de collage est un cathéter qui permet d'appliquer une colle en fibrine.

23. Instrument selon l'une des revendications 1 à 22,
**caractérisé en ce que** les mâchoires de pince (16) sont formées respectivement par deux mâchoires de préhension ou de cisaillement.

24. Instrument selon la revendication 23,
**caractérisé en ce que** les mâchoires de préhension sont pourvues de dents.

25. Instrument selon la revendication 23 ou 24,
**caractérisé en ce que** la forme des mâchoires de préhension est adaptée aux parties tissulaires qu'il s'agit d'assembler.

26. Instrument selon l'une des revendications 1 à 25,
**caractérisé en ce que** les mâchoires de pince (16) sont additionnellement susceptibles d'être déplacées en translation dans la direction de l'axe longitudinal de l'instrument.

27. Instrument selon la revendication 26,
**caractérisé en ce que** chaque mâchoire de pince (16) est déplaçable en translation indépendamment de l'autre mâchoire de pince (16).
